(19) Europäisches Patentamt / European Patent Office / Office européen des brevets

(11) **EP 3 468 460 B1**

(12) **EUROPEAN PATENT SPECIFICATION**

(45) Date of publication and mention
of the grant of the patent:
**26.08.2020   Bulletin 2020/35**

(21) Application number: **16904894.9**

(22) Date of filing: **12.06.2016**

(51) Int Cl.:
***A61B 5/01*** *(2006.01)*

(86) International application number:
**PCT/CN2016/085490**

(87) International publication number:
**WO 2017/214775 (21.12.2017 Gazette 2017/51)**

(54) **METHOD AND APPARATUS FOR ESTIMATING A BODY TEMPERATURE**

VERFAHREN UND VORRICHTUNG ZUR SCHÄTZUNG EINER KÖRPERTEMPERATUR

PROCÉDÉ ET APPAREIL D'ESTIMATION DE LA TEMPÉRATURE CORPORELLE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(43) Date of publication of application:
**17.04.2019   Bulletin 2019/16**

(73) Proprietor: **Nokia Technologies Oy
02610 Espoo (FI)**

(72) Inventors:
• **LAN, Peng
  Beijing 100023 (CN)**
• **LIANG, Yuyang
  Beijing 100088 (CN)**

(74) Representative: **Sayer, Robert David
Venner Shipley LLP
200 Aldersgate
London EC1A 4HD (GB)**

(56) References cited:
**CN-A- 101 404 928        CN-A- 104 068 832
CN-A- 104 586 407        CN-U- 203 576 480
US-A1- 2002 114 375      US-A1- 2007 239 038
US-A1- 2013 085 708      US-A1- 2014 323 826
US-A1- 2016 073 890      US-B1- 6 789 936**

**Description**

TECHNICAL FIELD

Embodiments of the present invention relate generally to body temperature measurement, more particularly, relate to a method and apparatus for estimating a body temperature.

BACKGROUND

Temperature is an important index of human health. If somebody's temperature is out of a normal scope such as 36.5-37.5 °C, that probably means an illness such as a flu, therefore people are used to measure body temperature for auto diagnosis. There are several ways to measure body temperature, for example, detecting human internal temperature such as that in the mouth, rectum or vagina or detecting tympanic temperature such as that in the ear, however the most popular and oldest way is to detect the armpit temperature with a traditional thermometer such as a mercury thermometer which is put under the closed armpit.

US-2002/114375-A1, US-6 789 936-B1, US-2016/073890-A and US-2013/085708-A1 show methods for estimating body core temperatures.

Modern thermometers such as a wearable thermometer which can be adhered to skin in order to acquire body skin temperature, and transfer temperature data directly to a receiving device such as a smartphone through wireless connections such as Bluetooth, WiFi etc.

Compared to traditional mercury thermometers, a wearable thermometer has several advantages:

1. A wearable thermometer can be attached onto any part of human body such as chest or abdomen, not requiring to be held tightly under the arm as a traditional thermometer, so it is easier and more convenient to use.

2. A wearable thermometer can continuously monitor temperature, for example, during sleep at night, and transfer temperature data to a receiving device such as a smart phone, iPad etc.

BRIEF SUMMARY

A method, apparatus and computer program product are therefore provided which may enable estimating a body temperature. In this regard, example embodiments of the present invention may obtain a receiving a measured body skin temperature and a measured environment temperature. Various embodiments may also estimate the body temperature based on the measured body skin temperature and the measured environment temperature by using the following equation:

$$Tb = f(Ts - Te) * (A + B*Ts + C*Te) + Ts$$

wherein, Tb denotes the body temperature, Ts denotes the measured skin temperature, Te denotes the measured environment temperature, A, B and C are parameters, f is a function such that f(x) is less than x when x is greater than 1.

In one example embodiment, a method for estimating a body temperature is described. The method may comprise receiving a measured body skin temperature and a measured environment temperature. The method may further comprise estimating the body temperature based on the measured body skin temperature and the measured environment temperature by using the following equation:

$$Tb = f(Ts - Te) * (A + B*Ts + C*Te) + Ts$$

wherein, Tb denotes the body temperature, Ts denotes the measured skin temperature, Te denotes the measured environment temperature, A, B and C are parameters, f is a function such that f(x) is less than x when x is greater than 1.

In another example embodiment, an apparatus for estimating a body temperature is described. The apparatus may comprise at least one processor, and at least one memory including computer program code. The at least one memory and the computer program code may be configured to, with the at least one the processor, cause the apparatus to receive a measured body skin temperature and a measured environment temperature. The at least one memory and the computer program code may be further configured to, with the at least one the processor, cause the apparatus to estimate the body temperature based on the measured body skin temperature and the measured environment temperature by using the following equation:

$$Tb = f(Ts - Te) * (A + B*Ts + C*Te) + Ts$$

wherein, Tb denotes the body temperature, Ts denotes the measured skin temperature, Te denotes the measured environment temperature, A, B and C are parameters, f is a function such that f(x) is less than x when x is greater than 1.

In another example embodiment, a wearable device configured to be worn a living body is described. The wearable device may comprise a first temperature sensor configured to measure a body skin temperature of the living body. The wearable device may further comprise a second temperature sensor configured to measure an environment temperature of the environment. The wearable device may further comprise the above apparatus in an example embodiment.

In another example embodiment, a computer program product for estimating a body temperature is described. The computer program product may comprise at least one computer-readable storage medium having computer-readable program code portions stored therein. The computer-readable program code portions may comprise first and second program code portions. The first program code portion may be configured to receive a measured body skin temperature and a measured environment temperature. The second program code portion may be configured to estimate the body temperature based on the measured body skin temperature and the measured environment temperature by using the following equation:

$$Tb = f(Ts - Te) * (A + B*Ts + C*Te) + Ts$$

wherein, Tb denotes the body temperature, Ts denotes the measured skin temperature, Te denotes the measured environment temperature, A, B and C are parameters, f is a function such that f(x) is less than x when x is greater than 1.

BRIEF DESCRIPTION OF THE DRAWING(S)

Having thus described the invention in general terms, reference will now be made to the accompanying drawings, which are not necessarily drawn to scale, and wherein:

Fig. 1 illustrates an example wearable device being attached to a living body and which may be used in embodiments of the invention;

Fig. 2 is a schematic block diagram of a wearable device according to example embodiments of the present invention;

Fig. 3 is a schematic diagram of a system for estimating a body temperature according to example embodiments of the present invention;

Fig. 4 is a schematic diagram of the apparatus for estimating a body temperature according to example embodiments of the present invention;

Fig. 5 is a flowchart of operations for estimating a body temperature according to example embodiments of present invention is provided.

DETAILED DESCRIPTION

A body skin temperature as measure by a wearable thermometer is lower than armpit temperature, so it is not suitable for medical usage.

In light of the above, it would be desirable to provide a new solution for estimating a body temperature, which can overcome the drawbacks of the prior solutions, and detect a body temperature continuously and at the same time accurately.

Embodiments of the present invention will now be described more fully hereinafter with reference to the accompanying drawings, in which some, but not all embodiments of the invention are shown. Indeed, the invention may be embodied in many different forms and should not be construed as limited to the embodiments set forth herein; rather, these embodiments are provided so that this disclosure will satisfy applicable legal requirements. Like reference numerals refer to like elements throughout. As used herein, the terms "data," "content," "information," and similar terms may be used interchangeably to refer to data capable of being transmitted, received and/or stored in accordance with embodiments of the present invention. Thus, use of any such terms should not be taken to limit the spirit and scope of embodiments of the present invention.

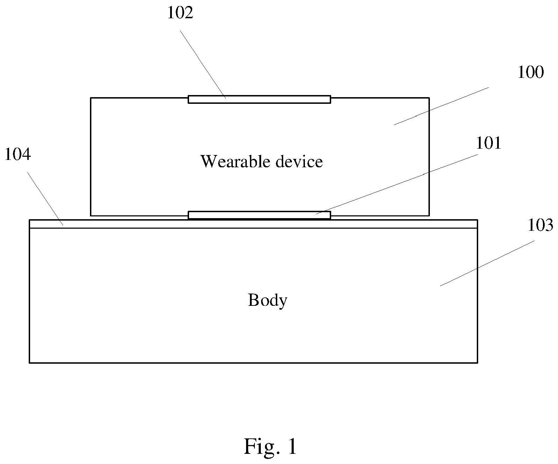

Referring now to Fig. 1, an example wearable device 100 being attached to a body 103 and which may be used in embodiments of the invention is illustrated. The wearable device 100 may be a wearable thermometer, or any other wearable device comprising temperature sensors, such as a smart watch, smart glasses, smart wrist band, smart bracelet, smart necklace, smart ankle bracelet, smart jewelry, earbud, activity tracker, smart clothing and accessories, wearable health monitoring or health care device, etc. with a temperature measuring function. As shown, the wearable device 100 may be adhered, fixed, stuck, bound, tied or otherwise attached to a body 103, such as a human body, and be kept in contact with the skin 104. The wearable device 100 may be attached to any part of the living body, for example, the chest, abdomen, wrist, etc.

The wearable device 100 may comprise at least two temperature sensors, for example, two temperature sensors: a first temperature sensor 101 and a second temperature sensor 102. The first temperature sensor 101 may be located at the bottom of the wearable device 100 to touch the skin for detecting the body skin temperature, and the second temperature sensor 102 may be located at the top of the wearable device 100 for detecting the environmental temperature. The temperature sensors may be any device or means which may be included in a wearable device and configured to detect temperature. For example, the temperature sensors may be thermostats, thermistors, resistive temperature detectors, thermocouples, or other types of temperature sensors. A thermal insulation layer may be located between these two temperature sensors in order to slow the temperature equilibrium between these two temperature sensors. The thermal insulation layer may be of any material, structure and size that may reduce heat transfer between the temperature sensors. In other embodiments, the wearable device 100 may comprise any other number of temperature sensors, provided only that both the body skin temperature and the ambient temperature may be measured.

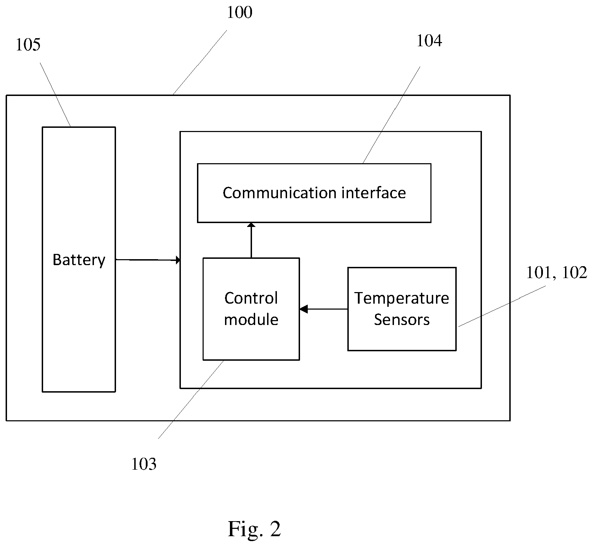

Referring to Fig. 2, a schematic block diagram of a wearable device 100 according to example embodiments of the present invention is provided. The wearable device 100 may be embodied as a chip or chip set. In other words, the wearable device 100 may comprise one or more physical packages (for example, chips) including materials, components and/or wires on a structural assembly (for example, a baseboard). The structural assembly may provide physical strength, conservation of size, and/or limitation of electrical interaction for component circuitry included thereon.

As shown, the wearable device 100 may comprise the temperature sensors 101 and 102 as described above, a control module 103, a communication module 104 and battery 105.

The control module 103 may receive data from the temperature sensors 101 and 102 and/or other sensors, perform processing on the data as needed, and send the data through the communication interface 104 to other devices for further processing, storage or presenting. The control module 103 may be embodied as any of various kinds of processing units, such as a micro controller unit (MCU), a microprocessor, etc.

The communication interface 104 may be used to send data to other devices. In some embodiments, the communication interface 104 may be a wireless communication interface, such as a Bluetooth Low Energy (BLE) interface, other wireless personal area network (WPAN) interface, WiFi interface, etc. Thus, the wearable device 100 may communicate wirelessly with other devices via a corresponding wireless communication technology.

The battery 105 may be used to supply power to the components of the wearable device 100, and may be any appropriate kind of battery, such as a lithium battery.

In some embodiments, the wearable device 100 may further comprise other components, such as a memory device for storing data to be processed and instructions configuring the control module 103, a digital processor for filtering the data received from the temperature sensors 101 and 102 and/or other sensors, an A/D converter for converting a analog signal sensed by the sensors to digital data, etc. These other components may be integrated in other components such as the control module 103 or may be separate components.

In some embodiments, the wearable device 100 may further comprise user interface and controls for receiving user input and presenting information to the user, for example, keys, a display, a speaker, etc. Moreover, the wearable device 100 may also comprise other sensors and functional components as needed for its specific functions.

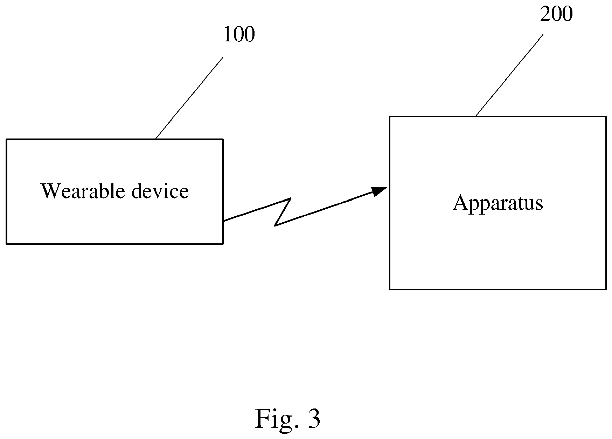

Referring to Fig. 3, a schematic diagram of a system for estimating a body temperature according to example embodiments of the present invention is provided. As shown, the system comprises the wearable device 100 as shown in Fig. 1 and described above, and an apparatus 200 for estimating a body temperature according to example embodiments of the present invention.

In example embodiments, the wearable device 100 may be in communication with the apparatus 200 for transmitting data such as temperature data measured by the temperature sensors to the apparatus 200. In some further example embodiments, the wearable device 100 may be configured to communicate wirelessly through its wireless communication interface with the apparatus 200 for transmitting the data.

In these example embodiments, the apparatus 200 may be any computing device or part thereof that may be configured to receive the data from the wearable device 100 via the wireless communication technology, perform the processing as described below on the data, and output the results of the processing, such as presenting to the user. In some embodiments, the apparatus 200 may be a smart mobile device, for example, a smart mobile phone, a tablet computer, etc, or part thereof. The apparatus 200 may be carried by the person who wears the wearable device 100, and thus, the apparatus 200 may be used to process the data transmitted from the wearable device 100 and present the results timely and conveniently to the person. Alternatively, the apparatus 200 may be carried by another person (for example, a parent, health care personnel, etc.) than the one (for example, a child, patient, etc.) who wears the wearable device 100, and thus, the apparatus 200 may be used to process the data transmitted from the wearable device 100 and present the results to the other person, for example, for parenting or health care purposes.

In some other example embodiments, the apparatus 200 may be integrated with, included in or embodied as the wearable device 100, and thus may receive the data such as temperature data measured by the temperature sensors via internal communication lines, perform the processing as described below on the data, and output the results of the processing, such as presenting to the user through an output interface of the wearable device 10, or sending to another computing device through an wireless communication interface of the wearable device 10.

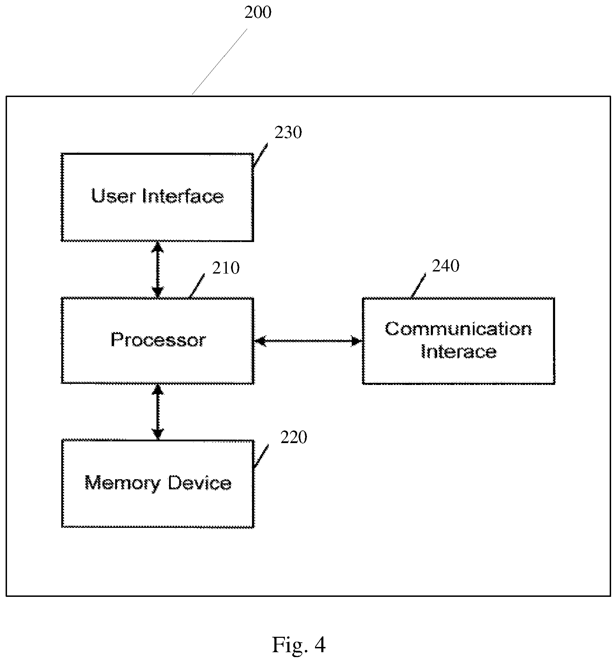

Referring to Fig. 4, a schematic diagram of the apparatus 200 for estimating a body temperature according to example embodiments of the present invention is provided. Apparatus 200 may comprises at least one processor 210, at least one memory device 220 including computer program code, an optional user interface 230 and/or an optional communication interface 240.

In an example embodiment, the processor 210 (and/or co-processors or any other processing circuitry assisting or otherwise associated with the processor 210) may be in communication with the memory device 220 via a bus for passing information among components of the apparatus 200. The memory device 220 may include, for example, one or more volatile and/or non-volatile memories. In other words, for example, the memory device 220 may be an electronic storage device (for example, a computer readable storage medium) comprising gates configured to store data (for example, bits) that may be retrievable by a machine (for example, a computing device like the processor 210). The memory device 220 may be configured to store information, data, applications, instructions, or the like for enabling the apparatus 200 to carry out various functions in accordance with an example embodiment of the present invention. For example, the memory device 220 could be configured to buffer input data for processing by the processor 210. Additionally or alternatively, the memory device 220 could be configured to store instructions for execution by the processor 210.

The apparatus 200 may be embodied as a chip or chip set. In other words, the apparatus 200 may comprise one or more physical packages (for example, chips) including materials, components and/or wires on a structural assembly (for example, a baseboard). The structural assembly may provide physical strength, conservation of size, and/or limitation of electrical interaction for component circuitry included thereon. The apparatus 200 may therefore, in some cases, be configured to implement an example embodiment of the present invention on a single chip or as a single "system on a chip." As such, in some cases, a chip or chipset may constitute means for performing one or more operations for providing the functionalities described herein.

The processor 210 may be embodied in a number of different ways. For example, the processor 210 may be embodied as one or more of various hardware processing means such as a coprocessor, a microprocessor, a controller, a digital signal processor (DSP), a processing element with or without an accompanying DSP, or various other processing circuitry including integrated circuits such as, for example, an ASIC (application specific integrated circuit), an FPGA (field programmable gate array), a microcontroller unit (MCU), a hardware accelerator, a special-purpose computer chip, or the like. As such, in an example embodiment, the processor 210 may include one or more processing cores configured to perform independently. A multi-core processor may enable multiprocessing within a single physical package. Additionally or alternatively, the processor 210 may include one or more processors configured in tandem via the bus to enable independent execution of instructions, pipelining and/or multithreading.

In an example embodiment, the processor 210 may be configured to execute instructions stored in the memory device 220 or otherwise accessible to the processor 210. Alternatively or additionally, the processor 210 may be configured to execute hard coded functionality. As such, whether configured by hardware or software methods, or by a combination thereof, the processor 210 may represent an entity (for example, physically embodied in circuitry) capable of performing operations according to an example embodiment of the present invention while configured accordingly. Thus, for example, when the processor 210 is embodied as an ASIC, FPGA or the like, the processor 210 may be specifically configured hardware for conducting the operations described herein. Alternatively, as another example, when the processor 210 is embodied as an executor of software instructions, the instructions may specifically configure the processor 210 to perform the algorithms and/or operations described herein when the instructions are executed. However, in some cases, the processor 210 may be a processor of a specific device (for example, a mobile terminal or network entity) configured to employ an example embodiment of the present invention by further configuration of the processor 210 by instructions for performing the algorithms and/or operations described herein. The processor 210 may include, among other things, a clock, an arithmetic logic unit (ALU) and logic gates configured to support operation of the processor 210.

Meanwhile, the optional communication interface 240 may be any means such as a device or circuitry embodied in either hardware or a combination of hardware and software that is configured to receive and/or transmit data from/to a network and/or any other device or module in communication with the wearable device 100. In this regard, the communication interface 240 may include, for example, an antenna (or multiple antennas) and supporting hardware and/or software for enabling communications with a wireless communication network. Additionally or alternatively, the communication interface 240 may include the circuitry for interacting with the antenna(s) to cause transmission of signals via the antenna(s) or to handle receipt of signals received via the antenna(s). In some environments, the communication interface 240 may alternatively or also support wired communication. As such, for example, the communication interface 240 may include a communication modem and/or other hardware/software for supporting communication via cable, digital subscriber line (DSL), universal serial bus (USB) or other mechanisms.

In an example embodiment, the apparatus 200 may include a user interface 230 that may, in turn, be in communication with the processor 210 to receive an indication of, or relating to, a user input and/or to cause provision of an audible, visual, mechanical or other output to the user. As such, the user interface 230 may include, for example, a keyboard, a mouse, a joystick, a display, a touch screen(s), touch areas, soft keys, a microphone, a speaker, or other input/output mechanisms.

Alternatively or additionally, the processor 210 may comprise user interface circuitry configured to control at least some functions of one or more user interface elements such as, for example, a speaker, ringer, microphone, display, and/or the like. The processor 210 and/or user interface circuitry comprising the processor 210 may be configured to control one or more functions of one or more user interface elements through computer program instructions (for example, software and/or firmware) stored on a memory accessible to the processor 210 (for example, memory device 220, and/or the like).

According to an example embodiment, communication interface 240 may be configured to communicate with a communication interface of the wearable device 100, either directly or over a network 200, for receiving data from the wearable device 100, and/or transmitting data and/or instructions to the wearable device 10.

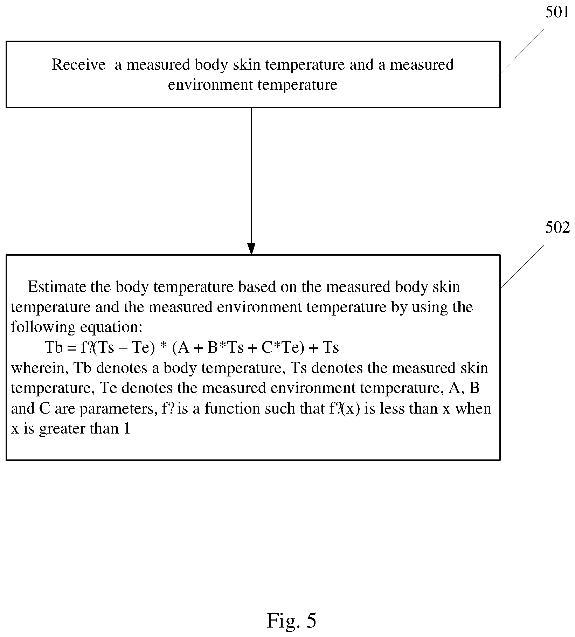

Referring to Fig. 5, a flowchart of operations for estimating a body temperature according to example embodiments of present invention is provided. The operations may be performed by the apparatus 200 according to example embodiments of the present invention, or may be performed by a computer program product for estimating a body temperature according to example embodiments of the present invention, or may constitute steps of a method for estimating a body temperature according to example embodiments of the present invention.

As shown in operation 501, the processor 210 of the apparatus 200 may be configured to receive a measured body skin temperature and a measured environment temperature. Specifically, the processor 210 may receive the measured body skin temperature and the measured environment temperature as measured by the first temperature sensor 101 and the second temperature sensor 102 of the wearable device 100 respectively from the wearable device 100.

As shown in operation 502, the processor 210 of the apparatus 200 may be further configured to estimate the body temperature based on the measured body skin temperature and the measured environment temperature by using the following equation:

$$Tb = f(Ts - Te) * (A + B*Ts + C*Te) + Ts, \qquad (1)$$

wherein, Tb denotes the body temperature, Ts denotes the measured skin temperature, Te denotes the measured environment temperature, A, B and C are parameters, f is a function such that f(x) is less than x when x is greater than 1. The estimated body temperature may be outputted, for example, presented to the user via a display device of the apparatus, or stored, transmitted or otherwise processed.

In the above equation, the requirement for the function f(x) to be less than x when x is greater than 1 is for the purpose of compensating the effect of a slower change of Te as compared to Ts in an initial phase of the wearable device's being activated. In such an initial phase, the temperatures Te and Ts increase fast, and Ts increases much faster than Te, so if a simple or quadratic equation of Ts and Te is used, then the estimated body temperature Tb would have a big jump in the initial phase. Use of a function f satisfying the above requirement may squeeze the gap between Ts and Te, so as to obtain a more reasonable result for all measurement phases.

Specifically, in some embodiments, the function f may be the SQRT function, that is, the above equation for estimating the body temperature may become:

$$Tb = SQRT(Ts - Te) * (A + B*Ts + C*Te) + Ts \qquad (2)$$

In some embodiments, the parameters A, B and C may be determined by fitting the equation to a set of experiment data on Tb, Ts and Te. That is, first a set of experiment data on Tb, Ts and Te of different people and in different environments may be collected, wherein the experiment data on Ts and Te may be collected by measuring the body skin temperatures and the environment temperatures using the wearable device 100 on different people in different environments, and the corresponding experiment data on Tb may be collected by measuring the body temperature using any device suitable for measuring the body temperature, such as a traditional thermometer put under the closed armpit. Then, the above equation (1) or (2) may be fitted to this set of experiment data to obtain the parameters A, B and C. Various function fitting methods, such as those known in the art, for example, the least squares method, etc., may be used to fit the function to the experiment data to obtain the parameters A, B and C.

In some embodiments, the parameters A, B and C may be determined respectively for multiple ranges of Ts by fitting the equation to the set of experiment data on Tb, Ts and Te in the multiple ranges of Ts respectively. That is, the set of experiment data on Tb, Ts and Te are first divided into multiple groups according to multiple ranges to which the value of Ts belongs, then the equation is fitted to the multiple groups respectively, so as to obtain multiple set of parameters A, B and C respectively. In this way, the fitting error may be reduced, and the parameters A, B and C may be fitted to the experiment data more accurately, thus obtaining an equation that can better estimate a body temperature Tb from a body skin temperature Ts and an environment temperature.

The number of ranges of Ts may be any appropriate number greater than two. The greater the number of ranges, the more accurate the fitting of the parameters A, B and C would be. In some embodiments, the number of ranges of Ts may be two, and the ranges of Ts may be, for example, Ts > 35.9 degrees Celcius and Ts < =35.9 degrees Celcius.

In an experiment conducted by the inventor, more than 100 sets of body and body skin temperature data of people and corresponding environment temperature data were collected, and by fitting the equation (2) to the sets of data divided into two ranges of Ts, Ts > 35.9 degrees Celcius and Ts < = 35.9 degrees Celcius, a set of parameters with A = 20.6137, B = 0.5419 and C = 0 for the range Ts < = 35.9 degrees Celcius, and a set of parameters with A = 8.6170, B = 0.2077 and C = 0 for the range Ts > 35.9 degrees Celcius, were generated. Further experiments showed that the equation (2) with these sets of parameters A, B and C has adequate accuracy in estimating a body temperature.

In some embodiments, the above operations 501 and 502 may be performed by the processor 210 continuously. Example embodiments of the present invention can estimate the body temperature accurately based on the body skin temperature and the environment temperature measured by a wearable device worn by a user, realizing both temperature measurement accuracy and continuous temperature monitoring.

As described above, Fig. 5 illustrates a flowchart of an apparatus 200, method, and computer program product for estimating a body temperature according to example embodiments of the invention. It will be understood that each block of the flowchart, and combinations of blocks in the flowcharts may be implemented by various means, such as hardware, firmware, processor, circuitry, and/or other devices associated with execution of software including one or more computer program instructions. For example, one or more of the procedures described above may be embodied by computer program instructions. In this regard, the computer program instructions which embody the procedures described above may be stored by a memory device 220 of an apparatus 200 employing an example embodiment of the present invention and executed by a processor 210 of the apparatus 200. As will be appreciated, any such computer program instructions may be loaded onto a computer or other programmable apparatus (for example, hardware) to produce a machine, such that the resulting computer or other programmable apparatus implements the functions specified in the flowchart blocks. These computer program instructions may also be stored in a computer-readable memory that may direct a computer or other programmable apparatus to function in a particular manner, such that the instructions stored in the computer-readable memory produce an article of manufacture the execution of which implements the function specified in the flowchart blocks. The computer program instructions may also be loaded onto a computer or other programmable apparatus to cause a series of operations to be performed on the computer or other programmable apparatus to produce a computer-implemented process such that the instructions which execute on the computer or other programmable apparatus provide operations for implementing the functions specified in the flowchart blocks.

Accordingly, blocks of the flowcharts support combinations of means for performing the specified functions and combinations of operations for performing the specified functions for performing the specified functions. It will also be understood that one or more blocks of the flowchart, and combinations of blocks in the flowchart, may be implemented by special purpose hardware-based computer systems which perform the specified functions, or combinations of special purpose hardware and computer instructions.

In an example embodiment, certain ones of the operations above may be modified or further amplified. Furthermore, in an example embodiment, additional optional operations may be included. Modifications, additions, or amplifications to the operations above may be performed in any order and in any combination.

Many modifications and other embodiments of the inventions set forth herein will come to mind to one skilled in the art to which these inventions pertain having the benefit of the teachings presented in the foregoing descriptions and the associated drawings. Therefore, it is to be understood that the inventions are not to be limited to the specific embodiments disclosed and that modifications and other embodiments are intended to be included within the scope of the appended claims. Moreover, although the foregoing descriptions and the associated drawings describe example embodiments in the context of certain example combinations of elements and/or functions, it should be appreciated that different combinations of elements and/or functions may be provided by alternative embodiments without departing from the scope of the appended claims. In this regard, for example, different combinations of elements and/or functions than those explicitly described above are also contemplated as may be set forth in some of the appended claims. Although specific terms are employed herein, they are used in a generic and descriptive sense only and not for purposes of limitation.

**Claims**

1. A method comprising:

   receiving a measured body skin temperature and a measured environment temperature; and
   estimating the body temperature based on the measured body skin temperature and the measured environment temperature by using the following equation:

$$Tb = f(Ts - Te) * (A + B*Ts + C*Te) + Ts$$

   wherein, Tb denotes the body temperature, Ts denotes the measured skin temperature, Te denotes the measured environment temperature, A, B and C are parameters, f is a function such that f(x) is less than x when x is greater than 1.

2. The method of claim 1, wherein f is the SQRT function.

3. The method of claim 1 or claim 2, wherein the parameters A, B and C are determined by fitting the equation to a set of experiment data on Tb, Ts and Te.

4. The method of claim 3, wherein the parameters A, B and C are determined respectively for multiple ranges of Ts by fitting the equation to the set of experiment data on Tb, Ts and Te in the multiple ranges of Ts respectively.

5. The method of claim 4, wherein the multiple ranges of Ts are the following two ranges of Ts: Ts > 35.9 degree Celcius, and Ts <= 35.9 degrees Celcius.

6. The method of any preceding_claim, wherein the measured body skin temperature and the measured environment temperature are measured by using a wearable thermometer with two temperature sensors for measuring the body skin temperature and the environment temperature respectively.

7. An apparatus (200) comprising

   at least one processor (210); and
   at least one memory (220) including computer program code;
   the at least one memory and the computer program code configured to, with the at least one processor, cause the apparatus to:

   receive a measured body skin temperature and a measured environment temperature; and
   estimate the body temperature based on the measured body skin temperature and the measured environment temperature by using the following equation:

$$Tb = f(Ts - Te) * (A + B*Ts + C*Te) + Ts$$

   wherein, Tb denotes the body temperature, Ts denotes the measured skin temperature, Te denotes the measured environment temperature, A, B and C are parameters, f is a function such that f(x) is less than x when x is greater than 1.

8. The apparatus of claim 7, wherein f is the SQRT function.

9. The apparatus of claim 7 or claim 8, wherein the parameters A, B and C are determined by fitting the equation to a set of experiment data on Tb, Ts and Te..

10. The apparatus of claim 9, wherein the parameters A, B and C are determined respectively for multiple ranges of Ts by fitting the equation to the set of experiment data on Tb, Ts and Te in the multiple ranges of Ts respectively.

11. The apparatus of claim 10, wherein the multiple ranges of Ts are the following two ranges of Ts: Ts > 35.9 degrees Celcius, and Ts < = 35.9 degrees Celcius.

12. The apparatus of any of claims 7 to 11, further comprising a communication interface for receiving the measured body skin temperature and the measured environment temperature from a wearable device with two temperature sensors for measuring the body skin temperature and the environment temperature respectively.

13. The apparatus of any of claims 7 to 12, wherein the apparatus is integrated with a wearable device with two temperature sensors for measuring the body skin temperature and the environment temperature respectively.

14. A wearable device configured to be worn a living body, comprising:

   a first temperature sensor configured to measure a body skin temperature of the living body;
   a second temperature sensor configured to measure an environment temperature of the environment; and
   the apparatus of any of claims 7 to 11.

15. A computer program product comprising at least one computer-readable storage medium having computer-readable program code portions stored therein, the computer-readable program code portions, when executed by a computer, causing the computer to carry out the method of any of claims 1 to 6.


**Patentansprüche**

1. Verfahren, umfassend:

   Empfangen einer gemessenen Körperhauttemperatur und einer gemessenen Umgebungstemperatur; und
   Schätzen der Körpertemperatur auf Basis der gemessenen Körperhauttemperatur und der gemessenen Umgebungstemperatur unter Verwendung der folgenden Gleichung:

$$Tb = f(Ts - Te)*(A + B*Ts + C*Te) + Ts,$$

   wobei Tb die Körpertemperatur bezeichnet, Ts die gemessene Hauttemperatur bezeichnet, Te die gemessene Umgebungstemperatur bezeichnet, A, B und C Parameter sind, f eine Funktion derart ist, dass f(x) kleiner als x ist, wenn x größer als 1 ist.

2. Verfahren nach Anspruch 1, wobei f die Quadratwurzelfunktion ist.

3. Verfahren nach Anspruch 1 oder Anspruch 2, wobei die Parameter A, B und C durch Anpassen der Gleichung an einen Satz von Versuchsdaten über Tb, Ts und Te bestimmt werden.

4. Verfahren nach Anspruch 3, wobei die Parameter A, B und C jeweils für mehrere Bereiche von Ts durch Anpassen der Gleichung an den Satz von Versuchsdaten über Tb, Ts und Te jeweils in den mehreren Bereichen von Ts bestimmt werden.

5. Verfahren nach Anspruch 4, wobei die mehreren Bereiche von Ts die folgenden beiden Bereiche von Ts sind: Ts > 35, 9 Grad Celsius und Ts <= 35, 9 Grad Celsius.

6. Verfahren nach einem vorhergehenden Anspruch, wobei die gemessene Körperhauttemperatur und die gemessene Umgebungstemperatur unter Verwendung eines Wearable-Thermometers mit zwei Temperatursensoren zum Messen der Körperhauttemperatur beziehungsweise der Umgebungstemperatur gemessen werden.

7. Vorrichtung (200), umfassend:

mindestens einen Prozessor (210); und
mindestens einen Speicher (220) mit Computerprogrammcode;
wobei der mindestens eine Speicher und der Computerprogrammcode ausgebildet sind, um mit dem mindestens einen Prozessor zu bewirken, dass die Vorrichtung ausführt:

Empfangen einer gemessenen Körperhauttemperatur und einer gemessenen Umgebungstemperatur; und
Schätzen der Körpertemperatur auf Basis der gemessenen Körperhauttemperatur und der gemessenen Umgebungstemperatur unter Verwendung der folgenden Gleichung:

$$Tb = f(Ts - Te) * (A + B*Ts + C*Te) + Ts,$$

wobei Tb die Körpertemperatur bezeichnet, Ts die gemessene Hauttemperatur bezeichnet, Te die gemessene Umgebungstemperatur bezeichnet, A, B und C Parameter sind, f eine Funktion derart ist, dass f(x) kleiner als x ist, wenn x größer als 1 ist.

8. Vorrichtung nach Anspruch 7, wobei f die Quadratwurzelfunktion ist.

9. Vorrichtung nach Anspruch 7 oder Anspruch 8, wobei die Parameter A, B und C durch Anpassen der Gleichung an einen Satz von Versuchsdaten über Tb, Ts und Te bestimmt werden.

10. Vorrichtung nach Anspruch 9, wobei die Parameter A, B und C jeweils für mehrere Bereiche von Ts durch Anpassen der Gleichung an den Satz von Versuchsdaten über Tb, Ts und Te jeweils in den mehreren Bereichen von Ts bestimmt werden.

11. Vorrichtung nach Anspruch 10, wobei die mehreren Bereiche von Ts die folgenden beiden Bereiche von Ts sind: Ts > 35, 9 Grad Celsius und Ts <= 35, 9 Grad Celsius.

12. Vorrichtung nach einem der Ansprüche 7 bis 11, weiterhin umfassend eine Kommunikationsschnittstelle zum Empfangen der gemessenen Körperhauttemperatur und der gemessenen Umgebungstemperatur von einer Wearable-Einrichtung mit zwei Temperatursensoren zum Messen der Körperhauttemperatur beziehungsweise der Umgebungstemperatur.

13. Vorrichtung nach einem der Ansprüche 7 bis 12, wobei die Vorrichtung mit einer Wearable-Einrichtung mit zwei Temperatursensoren zum Messen der Körperhauttemperatur beziehungsweise der Umgebungstemperatur integriert ist.

14. Wearable-Einrichtung, die ausgebildet ist, um an einem lebenden Körper getragen zu werden, umfassend:

einen ersten Temperatursensor, der ausgelegt ist zum Messen einer Körperhauttemperatur des lebenden Körpers;
einen zweiten Temperatursensor, der ausgebildet ist zum Messen einer Umgebungstemperatur der Umgebung; und
die Vorrichtung nach einem der Ansprüche 7 bis 11.

15. Computerprogrammprodukt umfassend mindestens ein computerlesbares Ablagemedium mit darauf gespeicherten computerlesbaren Programmcodeabschnitten, wobei die computerlesbaren Programmcodeabschnitte bei Ausführung durch einen Computer bewirken, dass der Computer das Verfahren nach einem der Ansprüche 1 bis 6 ausführt.

**Revendications**

1. Procédé comprenant :

la réception d'une température de peau corporelle mesurée et d'une température d'environnement mesurée ; et l'estimation de la température corporelle en fonction de la température de peau corporelle mesurée et de la température d'environnement mesurée au moyen de l'équation suivante :

$$Tb = f(Ts-Te)*(A+B*Ts+C*Te)+Ts$$

dans laquelle Tb désigne la température corporelle, Ts désigne la température de peau mesurée, Te désigne la température d'environnement mesurée, A, B et C sont des paramètres, f est une fonction telle que f(x) est inférieur à x quand x est supérieur à 1.

2. Procédé de la revendication 1, dans lequel f est la fonction racine carrée.

3. Procédé de la revendication 1 ou la revendication 2, dans lequel les paramètres A, B et C sont déterminés par ajustement de l'équation à un ensemble de données expérimentales sur Tb, Ts et Te.

4. Procédé de la revendication 3, dans lequel les paramètres A, B et C sont déterminés respectivement pour de multiples plages de Ts par ajustement de l'équation à l'ensemble de données expérimentales sur Tb, Ts et Te dans les multiples plages de Ts, respectivement.

5. Procédé de la revendication 4, dans lequel les multiples plages de Ts sont les deux plages suivantes de Ts : Ts > 35, 9 degrés Celsius, et Ts ≤ 35, 9 degrés Celsius.

6. Procédé d'une quelconque revendication précédente, dans lequel la température de peau corporelle mesurée et la température d'environnement mesuré sont mesurées au moyen d'un thermomètre portable avec deux capteurs de température destinés à mesurer la température de peau corporelle et la température d'environnement, respectivement.

7. Appareil (200) comprenant
au moins un processeur (210) ; et
au moins une mémoire (220) comportant un code de programme informatique ;
l'au moins une mémoire et le code de programme informatique étant configurés pour, avec l'au moins un processeur, conduire l'appareil à :

    recevoir une température de peau corporelle mesurée et une température d'environnement mesuré ; et
    estimer la température corporelle en fonction de la température de peau corporelle mesurée et de la température d'environnement mesuré en utilisant l'équation suivante :

$$Tb = f(Ts-Te)*(A+B*Ts+C*Te)+Ts$$

dans laquelle Tb désigne la température corporelle, Ts désigne la température de peau mesurée, Te désigne la température d'environnement mesuré, A, B et C sont des paramètres, f est une fonction telle que f(x) est inférieur à x quand x est supérieur à 1.

8. Appareil de la revendication 7, dans lequel f est la fonction racine carrée.

9. Appareil de la revendication 7 ou la revendication 8, dans lequel les paramètres A, B et C sont déterminés par ajustement de l'équation à un ensemble de données expérimentales sur Tb, Ts et Te.

10. Appareil de la revendication 9, dans lequel les paramètres A, B et C sont déterminés respectivement pour de multiples plages de Ts par ajustement de l'équation à l'ensemble de données expérimentales sur Tb, Ts et Te dans les multiples plages de Ts, respectivement.

11. Appareil de la revendication 10, dans lequel les multiples plages de Ts sont les deux plages suivantes de Ts : Ts > 35, 9 degrés Celsius, et Ts ≤ 35, 9 degrés Celsius.

12. Appareil de l'une quelconque des revendications 7 à 11, comprenant en outre une interface de communication destinée à recevoir la température de peau corporelle mesurée et la température d'environnement mesuré depuis un dispositif portable avec deux capteurs de température destinés à mesurer la température de peau corporelle et la température d'environnement, respectivement.

**13.** Appareil de l'une quelconque des revendications 7 à 12, l'appareil étant intégré à un dispositif portable avec deux capteurs de température destinés à mesurer la température de peau corporelle et la température d'environnement, respectivement.

**14.** Dispositif portable configuré pour être porté sur un corps vivant, comprenant :

un premier capteur de température configuré pour mesurer une température de peau corporelle du corps vivant ;
un deuxième capteur de température configuré pour mesurer une température d'environnement de l'environnement ; et
l'appareil de l'une quelconque des revendications 7 à 11.

**15.** Produit-programme informatique comprenant au moins un support de stockage lisible par ordinateur dans lequel sont stockées des parties de code de programme lisibles par ordinateur, les parties de code de programme lisibles par ordinateur, lorsqu'elles sont exécutées par un ordinateur, conduisant l'ordinateur à réaliser le procédé de l'une quelconque des revendications 1 à 6.

102

100

104

Wearable device

101

103

Body

Fig. 1

100

105

104

Battery

Communication interface

Control
module

Temperature
Sensors

101, 102

103

Fig. 2

100

200

Wearable device

Apparatus

Fig. 3

200

230

User Interface

210

Processor

240

Communication
Interace

220

Memory Device

Fig. 4

501

Receive a measured body skin temperature and a measured environment temperature

502

Estimate the body temperature based on the measured body skin temperature and the measured environment temperature by using the following equation:

Tb = f?(Ts − Te) * (A + B*Ts + C*Te) + Ts

wherein, Tb denotes a body temperature, Ts denotes the measured skin temperature, Te denotes the measured environment temperature, A, B and C are parameters, f? is a function such that f?(x) is less than x when x is greater than 1

Fig. 5

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2002114375 A1
- US 6789936 B1
- US 2016073890 A
- US 2013085708 A1